# EUROPEAN PATENT APPLICATION

(11) **EP 1 486 170 A1**
(43) Date of publication of application: **15.12.2004**
(21) Application number: 03425637.0
(22) Date of filing: 30.09.2003
(51) Int. Cl.: A61B 10/00

(54) **BIOPSY NEEDLE**

(30) Priority: 13.06.2003 IT PO20030007
(71) Applicant: Gironi, Aurelio, 59100 Prato (IT)
(72) Inventor: Gironi, Aurelio, 59100 Prato (IT)
(74) Representative: Martini, Lazzaro

(57) **Abstract**

Means for the consecutive drawing of two or more samples of organic material by means of biopsy needles on diagnostic purposes, characterised in that it introduces a new drawing technique. Said technique consists in drawing two or more samples while keeping said drawing means inside the body.

## Description

The present invention relates to a means for carrying out drawings by means of bioptic needle.

It is well known that drawings by means of bioptic needles imply the use of needles which hold tissue or organic samples drawn on human beings on diagnostic purposes.
These needles basically consist of a hollow and sharp cannula and of a stylet. During the path which the needle follows to reach the tissue to be analysed, the tip of the stylet juts out of the cannula, so as to prevent the tissue area, in which it moves forward, to penetrate into it. As soon as the two elements of the needle reach the area to be analysed, they move, so as trap a sufficiently large and integral sample, in order to carry out an histopatological test on its structure.

As the area in which the drawing takes place may be dishomogeneous, it may be necessary to draw two samples in order to better analyse its features. Therefore, before carrying out a second drawing, it is necessary to extract the needle from the body and replace it with a new one, after carrying out the first drawing.

The main object of the present invention is that of eliminating this inconvenience.

These results have been achieved in compliance with the invention, thanks to the idea of realising a needle having the features described in the independent claims. Further features are the subject of the dependent claims.

According to the present invention, it is possible to carry out two or more consecutive drawings by using the same needle, without extracting the needle from the body and replacing it with a new one. It is evident that, thanks to this technique, the traumatic effects of drawings by means of bioptic needles can be considerably reduced.

The technicians who work in this field can better understand the advantages and the features of the present invention thanks to the following description and to the enclosed drawings, which are a practical exemplification of the present invention, but should not be considered in a restrictive sense, in which:
- Figs 1A and 1B respectively show a longitudinal section view of a drawing means in compliance with the invention and a cross section view of said drawing means;
- Figs 1C, 1D and 1E respectively show the sequential motions of the stylet and of the cannula of the drawing means showed in Fig. 1A and 1B, while this drawing means is carrying out the drawing;
- Figs 2A and 2B consecutively show a longitudinal section view of a drawing means according to a further embodiment of the present invention and a cross section view of said drawing means;
- Fig 2C shows the final configuration of the drawing means showed in Figs 2A and 2B, after it has trapped three samples to be analysed;
- Figs 3A and 3B respectively show a longitudinal section view of a drawing means according to a further embodiment of the present invention and a cross section view of said drawing means;
- Fig. 3C shows the final configuration of the drawing means showed in Figs 3A and 3B, after it has trapped four samples to be analysed.

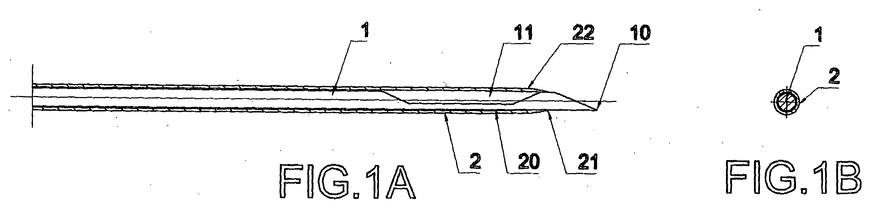

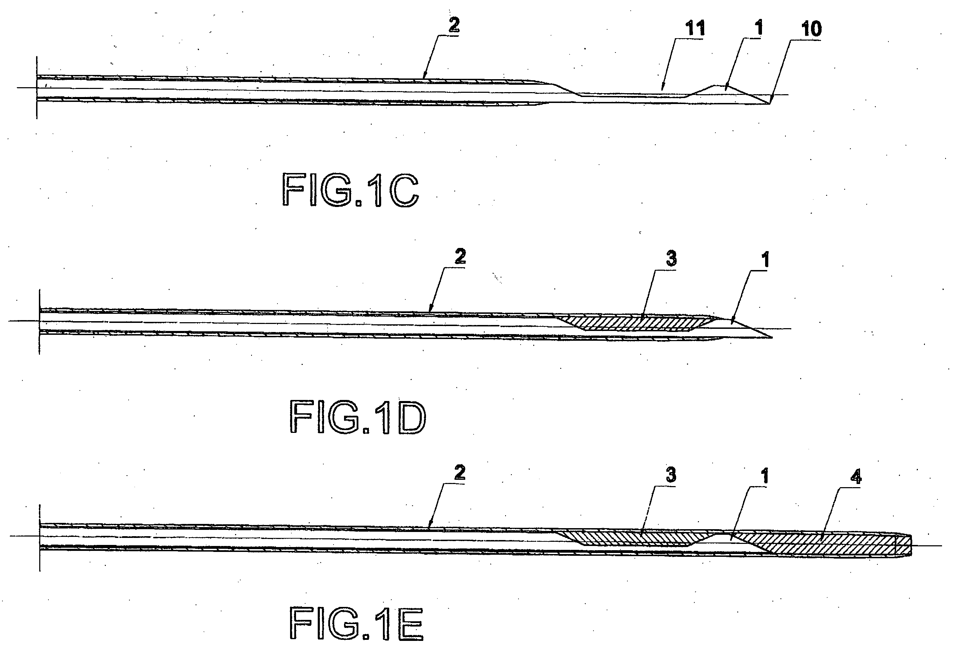

With reference to Figs 1A-1E of the enclosed drawings, the stylet (1) and the cannula (2), thanks to their structure, are capable of holding two tissue samples. The stylet (1), which features a cylindrical cross section, is provided with a sharp end (10) in proximity to which there is a cavity (11). The cannula (2), which features an internal cylindrical surface (20) whose diameter is large enough to allow the stylet to pass through it, has a sharp end (21) with a conical structure (2) which is capable of offering sufficient resistance to the exit of the material, once the material is inside the cannula.

The exit of the stylet (1) from the cannula (2) allows the adjacent tissue to adhere to its cavity (11), once these elements have reached the area to be analysed. The sliding of the cannula (2) on the stylet (1) in the direction in which it moves to approach its sharp end (10) without overtaking it, causes the cut of the first tissue sample (3), which remains isolated inside the needle. The further forward motion of the cannula (2) allows the drawing of a second sample (4) which is sheared and held inside surface (20) of the free end of stylet (1). As illustrated in the enclosed drawings, the above cavity defines a cradle in proximity to the end (10) of the stylet (1).

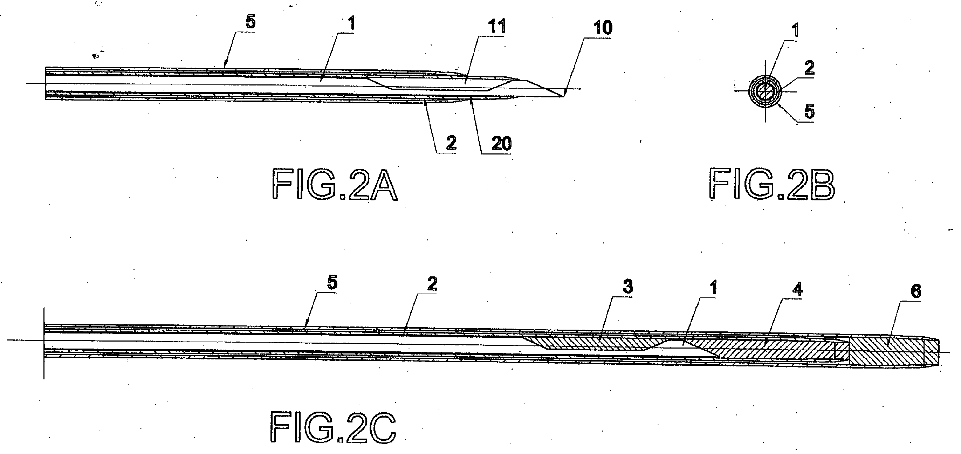

The examples in Figs 2A-2C of the enclosed drawings foresee a further embodiment of the present drawing means.
In particular, in comparison with the above example and with reference to Figs 1A-1B, we can see that a second cannula (5) has been added. The motions both of the stylet (1) and of the cannula (2) for drawing the first two samples are described in Figs 1C-1D-1E. The forward motion of the second cannula (5) allows the entrapment the third sample (6).

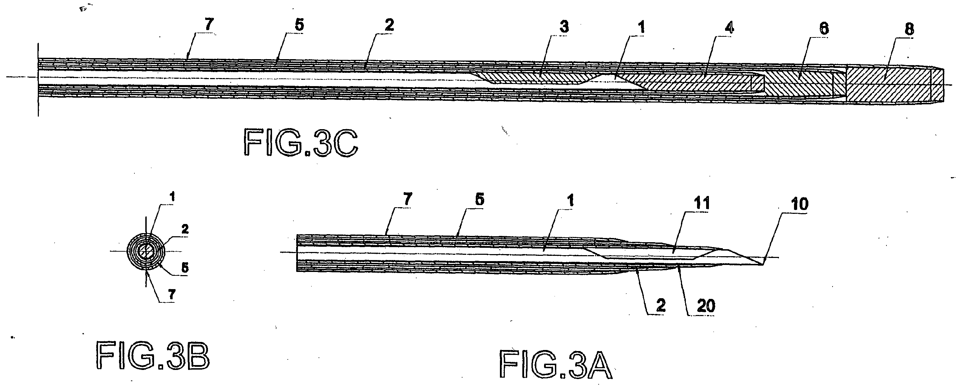

The examples illustrated in Figs 3A-3C of the enclosed drawings foresee a further embodiment of the present drawing means.
In particular, in comparison with the above example, with reference to Figs 2A-2B, we can see that a third cannula (7) has been added. The motions of stylet (1) of the first cannula (2) and of the second cannula (5) are the same described in the example showed in Figs 2A-2B-2C.
The forward motion of the third cannula (7) allows the entrapment of a fourth sample (8).

## Claims

1. Means for drawing organic material **characterised in that** it consists of elements for drawing two or more material samples.

2. Means according to claim (1) **characterised in that** it comprises two or more active elements, that is movable towards the tissue or the organ from which remove two or more samples.

3. Means according to claim 1 or 2 **characterised in that** it comprises elements which isolate the entrapped tissue samples from the tissue adjacent to said means.

4. Means for drawing organic material according to one or more previous claims, **characterised in that** it comprises a stylet (1) having a cylindrical cross section with a sharp end (10) in proximity to which there is a cavity (11); a cannula (2) featuring an internal cylindrical surface (20) whose diameter is sufficiently large to allow the stylet to slide in its interior, as well as a sharp end (21) whose conical structure (22) offers sufficient resistance to the exit of the material, once the latter has penetrated into it, and in which the above cavity (11) defines a crandle in proximity to said end (10) of stylet (1).
